# EUROPEAN PATENT SPECIFICATION

(11) **EP 0 498 382 B1**
(45) Date of publication and mention of the grant of the patent: **13.11.1996**
(21) Application number: 92101866.9
(22) Date of filing: 05.02.1992
(51) Int. Cl.: A61M 1/28

(54) **Continuous peritoneal dialysis system**
System zur kontinuierlichen Peritonaldialyse
Système de dialyse péritonéale continue

(30) Priority: 08.02.1991 US 653078
(43) Date of publication of application: 12.08.1992
(73) Proprietor: Peabody, Alan M., Greer South Carolina 29650 (US)
(72) Inventor: Peabody, Alan M., Greer, South Carolina 29650 (US); Boag, James T., Seattle, Washington 98112 (US); Walters, Ted M., Anderson, South Carolina 29621 (US)
(74) Representative: Neubauer, Hans-Jürgen, Dipl.-Phys.

(56) References cited:
- EP-A- 0 243 547
- EP-A- 0 402 505
- DE-A- 3 121 098
- FR-A- 2 440 740
- GB-A- 2 091 126
- US-A- 4 190 047

## Description

### Background of the Invention

The invention relates to a dialysis system for purifying the blood by exchange across the peritoneal membrane during a continuous peritoneal process.

Heretofore, artificial kidney users have relied basically on two processes for purifying the blood. Hemodialysis involves the circulation of blood through a dialysis machine in which an exchange of toxic metabolites takes place across an artificial membrane outside the patient's body. This process requires the assistance of trained personnel and subjects the patient to dangers of mechanical malfunction, and rapid shifts of fluid and metabolites, and surgery involving blood vessels, is involved.

Peritoneal dialysis involves the installation of a sterile dialysate into the peritoneal cavity. The dialysate is discarded after absorbing waste metabolites. The process is repeated until the blood level of metabolites is reduced to a desired level. This method is commonly referred to as the "Batch" method due to the fact that numerous one or two liter bottles or bags of fresh dialysate solution are utilized, requiring multiple connections to be made to the catheter inserted in the peritoneal cavity. The multiple connections made during the course of the dialysis have been thought to be a major cause of the high instance of peritonitis.

Chronic ambulatory peritoneal dialysis offers peritoneal dialysis while still allowing the patient freedom of movement. However, the chronic ambulatory peritoneal dialysis must be done in absence of a machine. Multiple bottles or bags of dialysis must be infused daily. The bag of dialysate is worn by the patient. The multiple installations per day require that multiple connections of bags or bottles to the peritoneal catheter be made. The production of bulk sterile dialysis for the peritoneal process has not been shown to be practical for large scale application particularly for home dialysis.

United States Patent No. 4,311,587 seeks to avoid some of the above problems with peritoneal dialysis by providing a sub-micron filter on line with the fresh dialysate to prevent peritoneal contamination. The system is perambulatory and the bag of dialysate is worn by the patient. The bag may be pressurized by numerous methods and is connected only to the inflow side of the filter. The outflow port of the filter is connected on the other side of the filter so that no peritoneal contaminating source is connected directly to the peritoneal cavity. The system is still basically a batch type system in that multiple bottles or bags of dialysate must be connected to the filter even though direct connection to the peritoneal catheter is not required.

United States Patent No. 4,338,190 discloses a system and process which attempts to avoid the batch process method utilized heretofore in peritoneal dialysis wherein a closed loop peritoneal circuit is provided having a selective membrane across which toxic metabolites are exchanged. A solution is passed on the other side of the selective membrane to maintain the original concentration of sugar and salt in the peritoneal fluid as the toxic metabolites pass the separator membrane. A concentrate of sugar and salts is mixed at a desired ratio with water to make up the dialysis fluid. The conductivity of the fluid may be automatically monitored to adjust the concentration of the fluid during its recirculation. A double peritoneal catheter provides for the inflow and outflow of the peritoneal fluid. The peritoneal fluid is constantly recirculated through the peritoneal cavity. The efficiency of the dialysis becomes reduced slightly because of residual toxins which are put back into the peritoneal cavity. The selective membrane is an expensive disposable item which means that the cost of operating the system is high unless the membrane is recleaned. Published European Application No. EP-A-0243547 discloses a peritoneal dialysis system wherein the inflow and outflow of dialysis fluid to the peritoneal cavity take place through a double catheter or a single catheter arrangement. However, the volume of fluid in the peritoneal cavity is maintained in a generally constant manner. The inflow and outflow are controlled so that a pool or volume of dialysis fluid in the cavity is generally constant. There is no tidal fluctuation of the volume of dialysis fluid in the peritoneal cavity during the dialysis regime followed by complete drainage of dialysis fluid and ultrafiltrate at the end of the regime. Pumping the peritoneal fluid through the peritoneal cavity is required making it difficult to assure that the patient stays properly distended during the dialysis process. If the peritoneal membrane is not fully distended, it becomes convoluted around the intestines and pockets are formed where the peritoneal fluid can sequester. Incomplete circulation results in decreased efficiency of dialysis. No control is maintained over the level of peritoneal fluid in the peritoneal circuit. There is no way of replenishing the peritoneal fluid should the circuit run low on fluid or run dry.

United States Patent No. 3,545,438 discloses a peritoneal dialysis method which provides for partially reusing a portion of the dialysis fluid. The spent fluid becomes mixed with fresh dialysate. There is no control or monitoring of the fluid in and out as would allow the dialysate to flow in the peritoneal cavity in a continuous manner.

United States Patent No. 3,707,967, corresponding to German Patent Nr. 2.149,040, apparently discloses a closed loop dialysis system wherein the dialysis fluid is continuously circulated through the peritoneal membrane, generally without flow control and monitoring of inflow and outflow lines in a continuous manner.

United States Patent No. 3,709,222 discloses a peritoneal dialysis method generally of the batch type. The amount of dialysis fluid entering the peritoneal cavity and the pressure of the peritoneal cavity are controlled by varying the height of a pressure relief chamber. This can be both inaccurate and unreliable, and requires that an attendant be present in order to carry out the process. The cycles involved in the process are rather complicated involving an initial priming cycle which involves the filling of a proportion chamber, the pressure relief chamber and a return chamber. To begin the process, dialysis fluid is allowed to enter the peritoneal cavity from the pressure relief chamber until the chamber is moved to a lower position which prevents the fluid from flowing. Next, an automatic cycling begins in which the fluid is pumped out of the cavity into the return chamber. Next, an inflow cycle begins in which the fluid is pumped from the return chamber to the proportional chamber which forces fresh dialysis from the proportioning chamber into the pressure relief chamber from where it flows into the patient. Next, there is an equilibrium cycle in which dialysis fluid is drained from the proportioning chamber and fresh dialysis fluid is added. Apparently, during this time, dialysis fluid remains in the cavity. The process then switches back to the outflow cycle in which fluid is pumped from the cavity to the return chamber.

French Patent No. 2,371,931 discloses a peritoneal process having a single line used for inflow and outflow. The method includes pumping in a certain amount of dialysate, letting it equilibrate for a prescribed dwell time, and removing the dialysate. This is, in essence, an automatic "batch" system.

United States Patent No. 4,412,917 discloses a peritoneal dialysis system wherein a prescribed volume of fluid is pumped into the peritoneal cavity and drained. The installation of fluid to the patient can be controlled by counting pump revolutions or pumping time and by monitoring the total weight of the reservoirs which contain the source and drain fluids to achieve a desired amount of fluid received by the patient. After the fluid is drained, the patient's fluid balance is determined by weighing the increase and total weight of fluid on output scales to indicate the extra amount of fluid (ultrafiltrate) received from a patient. The fill and drain cycles may be repeated until either a desired amount of fluid is used or the change in fluid balance reported by the scales is of a desired magnitude.

It is also known in peritoneal dialysis to use a reverse osmosis unit to supply water which is passed through a heat exchanger for heating. The water is subsequently mixed With a dialysis concentrate, and optionally, dextrose, which are mixed in a predetermined ratio to provide a dialysate. The dialysate may be gravity fed or pumped to the patient, and thereafter drained either by gravity or pumping in a semi-automatic manner. However, the system is complex for the patient to operate, and has difficulty in meeting safety requirements. It is also known to mix dextrose with the water and dialysis concentrate to effect the osmolality over pressure sensors are utilized to sense an excessive pressure condition in the peritoneal cavity.

Tidal peritoneal dialysis is a technique where, after an initial fill of the peritoneal cavity, less than 50% of dialysate is drained and replaced by fresh dialysis fluid with each cycle, leaving the majority of the dialysate in constant contact with the peritoneal membrane until the end of the dialysis session when the fluid is drained as completely as possible. The factors determining the efficiency of dialysis with the tidal technique include the minimum volume of fluid in the peritoneal cavity assuring constant and full contact between the peritoneal membrane and dialysate, that is the reserve volume, and proper mixing of fluid in the peritoneal cavity by a sufficiently high tidal exchange volume. While the technique of tidal peritoneal dialysis is sound and efficient, there is a lack of suitable systems for implementing the tidal technique of peritoneal dialysis in a practical manner. None of the prior peritoneal dialysis methods and systems described previously are entirely suitable.

Accordingly, an important object of the present invention is to provide a continuous peritoneal dialysis system which avoids the inherent problems and dangers of a batch type peritoneal dialysis system.

Still another important object of the present invention is to provide a peritoneal dialysis system having a high rate of dialysate exchange providing increased dialysis efficiency.

Still another important object of the present invention is to provide a peritoneal dialysis system having a high rate of dialysate exchange and dialysis efficiency in which the osmolality of the fluid is continuously adjusted in response to the amount of fluid removed from the patient.

Still another important object of the present invention is to provide a continuous peritoneal dialysis system which may be used to carry out a continuous peritoneal dialysis using a tidal dialysis regime in the patient's peritoneal cavity wherein a fluctuating volume of dialysis fluid is maintained in the cavity during repeated drain and fill cycles followed by a complete drain of dialysis fluid and ultrafiltrate at the end of the regime.

Yet another important object of the present invention is to provide a continuous flow peritoneal dialysis system in which the pressure and volume of dialysate in the patient's peritoneal membrane may be monitored without the need of a medical attendant during the tidal dialysis regime.

### Summary of the Invention

The above objectives are accomplished according to the present invention by a system for performing a continuous peritoneal dialysis process which comprises a virtually unlimited supply of sterilized dialysis fluid, and an inflow delivery value for filling the peritoneal cavity with dialysis fluid through a catheter from the supply source. An outflow delivery value controls draining the dialysis fluid from the peritoneal cavity. An inflow measuring meter monitors a function of an inflow of dialysis fluid which fills the peritoneal cavity through the inflow catheter and generates an inflow signal representing the amount of fluid delivered into the peritoneal cavity. An outflow measuring meter monitors a function of an outflow of dialysis fluid drained through the outflow catheter and generates an outflow signal representing the amount of fluid drained from the peritoneal cavity.

A control computer controls the inflow delivery value to control the amount of dialysis fluid delivered to the peritoneal cavity in response to the inflow signal, and controls the outflow delivery value to control the amount of dialysis fluid drained from the peritoneal cavity in response to the outflow signal.

Sterilized dialysis fluid may be accumulated in a first reservoir. A first weighing cell weighs the dialysis fluid in the first reservoir to accumulate a first prescribed volume of dialysis fluid in said the first reservoir and generates a first weight signal. The inflow delivery value delivers the dialysis fluid from the first reservoir to the peritoneal cavity of a patient. The outflow delivery means for draining dialysis fluid from the peritoneal cavity of the patient into a second reservoir. A second weighing cell weighs the dialysis fluid in the second reservoir to determine a second prescribed volume of dialysis fluid and generates a second weight signal. The control computer may also control the inflow delivery value to terminate filling of the peritoneal cavity in response to the first weight signal and inflow signal, and control the outflow delivery value to terminate the draining of the dialysis fluid from the peritoneal cavity in response to the second weight signal and the outflow signal.

There is a monitor for monitoring a function of the fluid volume in the peritoneal cavity for controlling the outflow delivery value to adjust the amount of dialysis fluid in the peritoneal cavity to maintain a desired fluid volume in the peritoneal cavity. The monitor may be an ultrasonic sensing or a resistive sensing device.

The control computer may control the inflow and the outflow delivery values for filling and draining the dialysis fluid so as to maintain a desired reserve volume of dialysis fluid in the peritoneal cavity which is effective for dialysis. The inflow and outflow delivery values may be controlled to initially fill the peritoneal cavity with the first prescribed volume of dialysis fluid, drain the dialysis fluid from the peritoneal cavity to leave a reserve volume of dialysis fluid remaining in the cavity which is less than the first prescribed volume, and fill the peritoneal cavity with a tidal volume of dialysis fluid. The control computer then repeats draining of the peritoneal cavity to the reserve volume and filling of the peritoneal cavity with the tidal volume for a prescribed number (N) of cycles of total volume. The control computer controls the inflow and outflow delivery values for totally draining the dialysis fluid from the peritoneal cavity at the end of N cycles. The control then repeats an initial filling of the peritoneal cavity with dialysis fluid, and drains the peritoneal cavity to the reserve volume and fills the peritoneal cavity with the tidal volume again for a prescribed number (N) of cycles, or total exchange volume.

A heater heats the dialysis fluid prior to accumulating the first volume of dialysis fluid in the first reservoir to sterilize the dialysis fluid. A storage stores the dialysis fluid at a prescribed temperature and for a prescribed length of time prior to delivering the dialysis fluid to the first reservoir to kill the bacteria in the dialysis fluid. A deaerator deaerates the dialysis fluid prior to accumulating the dialysis fluid in the first reservoir. A detector detects the conductivity of the dialysis fluid prior to delivering the dialysis fluid to the first reservoir for maintaining a prescribed conductivity. A sensor senses the amount of water removed from the patient during dialysis and adjusts an amount of glucose in the dialysis fluid to adjust the osmolality and maintain a prescribed weight removal from the patient.

### Description of the Drawings

The construction designed to carry out the invention will hereinafter be described, together with other features thereof. The invention will be more readily understood from a reading of the following specification and by reference to the accompanying drawings forming a part thereof, wherein an example of the invention is shown and wherein:

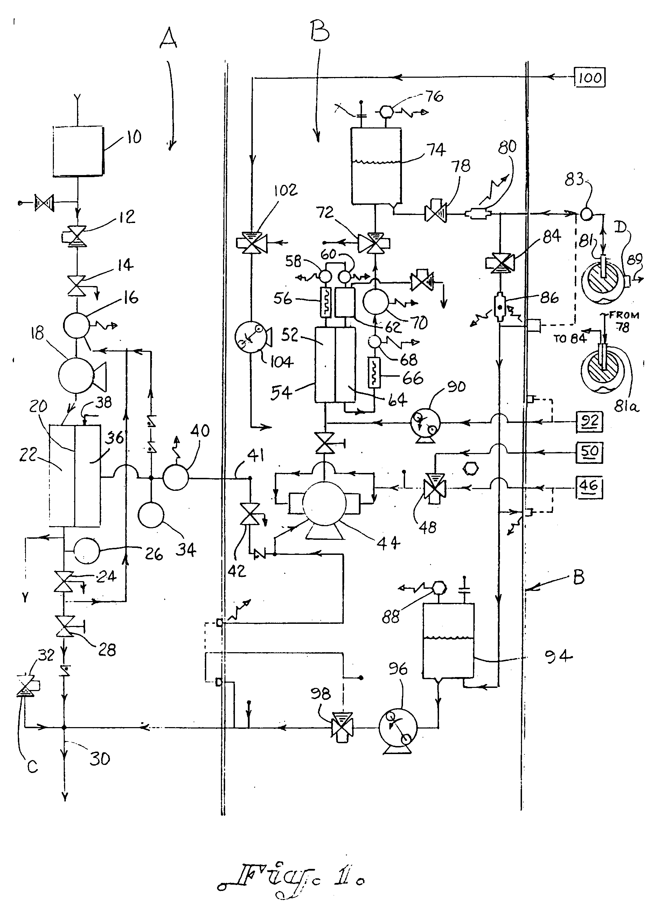

Figure 1 is a schematic diagram of a peritoneal dialysis system according to the invention;
Figure 2 is a graph illustrating a ready cycle of a sterilization system for a dialysis system according to the invention;
Figure 2-A is a graph illustrating a regime for a peritoneal tidal dialysis system according to the invention;
Figure 2-B is a graph illustrating a total drain cycle for a peritoneal tidal dialysis system according to the invention;
Figure 2-C is a graph illustrating a sterilization cycle for a peritoneal dialysis system according to the invention;
Figure 3 is a schematic diagram of a computer controller for a peritoneal dialysis system according to the invention; and
Figure 4 is a graph of a peritoneal dialysis system according to the invention using a tidal dialysis regime illustrating the buildup of ultrafiltrate during the tidal regime.

### Description of a Preferred Embodiment

Referring now in more detail to the drawings, an illustrative embodiment of the invention will now be described. There is a reverse osmosis (RO) unit A to which water is delivered through an input filter 10 which may consist of a carbon element to remove chlorine and chloramines followed by a particle filter. A suitable RO unit is manufactured by Servall Corporation of Anderson, South Carolina, as model 2114B. There is a main water valve 12 which is always on except in the recirculating sterilize mode. Next comes a manual pressure regulator 14 which essentially sets the product water pressure. A conductivity/temperature (C/T) probe 16 measures the incoming water conductivity and temperature and signals the electronics of RO unit A. The word "conductivity" is common in water treatment systems, and the term "TDS" as used herein stands for total dissolved solids. Pump 18 is the main RO water pump and supplies the flow and pressure to drive water across a RO membrane 20 of RO unit A. After passing through an inlet side of the RO membrane case 22 the water flow normally enters a second manual pressure regulator 24, monitored by a pressure gauge 26 which sets the membrane inlet pressure in the range of 150 to 175 psi. This is followed by a manually adjusted fluid restrictor 28 (and check valve) which adjusts the amount of water recirculating in the loop back to the connection point of probe 16. The waste water then flows down a drain 30. A valve 32 is actuated momentarily by a computer C at the beginning of a READY/RINSE cycle to allow a high flow of water through to flush inlet side 22 of membrane 20. The pressure in the output product water is monitored by a gauge 34. Note that an outlet side 36 of the membrane housing includes a formaldehyde inlet 38 where formaldehyde is introduced during a STERILIZATION. A probe 40 measures the conductivity and temperature of the product water and signals the RO electronics. The RO electronics watch both the ratio of the incoming and product water conductivity (% rejection) and the output conductivity and communicate with the computer. Both the output conductivity and the % rejections have alarm limits set by the RO electronics which signal the computer. There is a loop back from a point prior to probe 40 through two check valves which essentially diverts flow back to the connection point at probe 16 to regulate the product water pressure.

The sterile product water at 41 passes into a dialysis production unit (DPU) module B. Production unit B includes a manually set pressure regulator 42 which controls the flow rate through a check valve to a volumetric proportioning pump 44. Pump 44 precisely proportions a concentrate 46 through a valve 48 and water through 42 to make dialysate. It also proportions bleach 50 at the start of a STERILIZE/BLEACH cycle. The output of pump 44 passes through a manually set fluidic restrictor to the heat exchanger input. The proportioning pump 44 may be in the range of a 34:1 to 20:1 to allow use of available peritoneal dialysis concentrate formulations. After passing through an inlet side 52 of a heat exchanger 54, the dialysate is heated by a heater means 56, controlled by thermistor 58 and the computer, to a temperature of 70° to 80° C and monitored by thermistor 60 and the computer. A storage means in the form of a small reservoir 62 following thermistor 60 serves two purposes, holding the fluid at that temperature for a short time to provide backup sterilization, and providing a point for air removal. In summary, heater 56 serves the dual purpose of backup sterilization and air removal. The dialysate then flows back through an opposite side 64 of heat exchanger 54 in a counterflow direction and is cooled below a heater 66 temperature set point. Heater 66 heats the dialysate to the proper patient temperature and is controlled by thermistor 68 and the computer. C/T probe 70 monitors both the temperature and conductivity of the dialysate. The computer receives the output of probe 70 and by controlling a valve 72 allows flow into a reservoir 74 only when both conductivity and temperature are within preset alarm limits. Otherwise the dialysate is diverted to drain.

The amount of fluid in reservoir 74 is precisely measured by first weighing means which includes a load cell 76 which generates a first weight signal 76a input to computer C. When the amount of fluid in reservoir 74 is correct and the cycle time is correct, the computer opens an inflow delivery means, in the form of a reservoir valve 78, to allow fluid to enter (FILL) the peritoneal cavity 81 of a patient through a flowmeter 80. Flowmeter 80 provides an inflow monitoring means which monitors a function of fluid inflow, i.e. flow rate, accumulation, etc., to measure the amount of fluid delivered into the peritoneal cavity, and generate an inflow signal 80a. The pulse output of flowmeter 80 may be totaled by computer C as a monitor or backup to the measurement by the load cell/computer system. An optional filter, such as a suitable micro pore filter, may be employed in the flow path at 83 to sterilize the dialysis fluid in the event a sterilized source is not used. After an optional dwell time (DWELL) the fluid is drained (DRAIN) by an outflow delivery means having a valve 84 opened by computer C. Note that the "A" port of valve 84 is connected to a manual sampling valve which is used to check the formaldehyde residual concentration. The drain fluid (which includes any ultrafiltrate) then passes through a flowmeter 86 which acts in a manner similar to flowmeter 80 as a backup/monitor to the fluid measurement of a load cell 88. Flowmeter 86 provides an outflow monitoring means for monitoring the outflow of fluid from the peritoneal cavity and generates an outflow signal 86a representing the amount of fluid drained which is transmitted to the computer. Flowmeters 80 and 86 may be any suitable sterilizable digital flowmeter such as an Opflowmeter manufactured by Headland Company of Racine, Wisconsin.

Preferably, the peritoneal cavity volume is also measured by a peritoneal monitor means D, preferably either in the form of a sonic or strain band system as an additional measurement of the fluid volume in the cavity to prevent over distension. Monitor means D generates a peritoneal signal 89 which represents the volume of fluid in the peritoneal cavity at a given moment. Peritoneal signal 89 can be used by computer C to override the inflow and outflow signals, 80a and 86a, and/or the weight signals, 76a and 88a, to maintain a desired or reserve amount of fluid in the peritoneal cavity at all times.

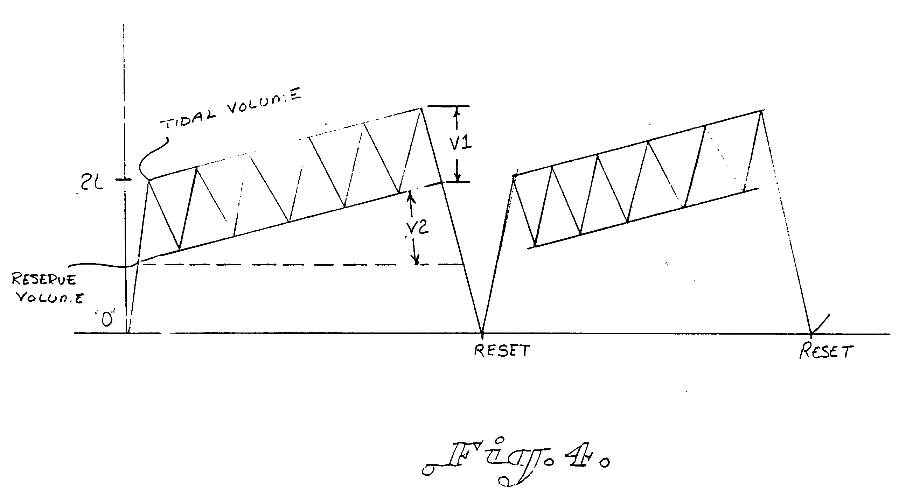

Sensor means for monitoring the amount of fluid removed from the patient and adjusting the osmolality of the dialysis fluid includes a dextrose pump 90 used to add dextrose 92 in addition to that contained in the peritoneal dialysis concentrate. In the tidal dialysis regime, according to the invention, a total drain occurs at the end of either a desired number (N) cycles, or time or volume of fluid which correspond to a desired number (N) of tidal fill and drain cycles. After the total drain, the total amount of water taken off the patient may be calculated by the computer and the osmolality of the solution adjusted to insure an accurate amount of ultrafiltrate is removed from the patient. As can be seen in Figure 4, the volume of fluid in the peritoneal cavity during the tidal peritoneal dialysis process is more accurately illustrated wherein volume V1 represents the tidal drain volume and volume V2 represents the ultrafiltration volume. Thus, even though the tidal drain volume V1, which may be 1 liter, for example, is being removed, the volume of fluid in the peritoneal cavity may be increasing due to the presence of the ultrafiltration. By having a total drain of the peritoneal cavity after a desired number of drain and fill cycles, it is possible to remove the ultrafiltration and begin the dialysis process again. Referring to Figure 4, the process begins at the origin where the peritoneal cavity volume is zero. The cavity is filled with a volume of 2 liters and then drained with a volume of 1 liter, as shown in the example of Figure 4. Because there may be some ultrafiltrate, the draining of 1 liter will not necessarily result in a reserve volume of 1 liter. The ultrafiltrate will increase this volume as shown at V2. In accordance with the invention, at the reset point there is a total drain, the amount of ultrafiltrate is measured, and the remaining weight to be removed from the patient is calculated. If the dialysis process is not removing weight at a sufficient rate, the osmolality of the dialysis solution may be adjusted by increasing the dextrose amount so that more weight is removed from the patient during the next dialysis regime consisting of tidal fill and drain cycles. If too much weight has been removed during the previous dialysis regime, then the dextrose can be decreased. At the beginning of the dialysis process, the dry weight of the patient has been calculated along with the specific amount of weight desired to be removed from the patient. It may be desirable to increase the maximum flow rate of pump 90 from 10 m./min (2%) to 15 m./min (3%) since concentrate dextrose amounts over 1.8% (diluted) tend to be unstable. The actual ultrafiltrate may be measured by calculating the amount of patient fluid removal, and pump 90 automatically adjusted to obtain the desired ultrafiltration amount. There is no backup monitor on pump 90. This may be considered a non-critical condition or might be monitored roughly by probe 70 since the dextrose decreases the conductivity.

The drain fluid enters a drain reservoir 94 where the amount is precisely monitored by a second weighing means which includes a load cell 88 which generates a second weight signal 88a input to the computer. The second weight signal represents an amount that includes both the fill volume and the ultrafiltrate and is stored by the computer. Note that the readings and signals obtained from weighing means 76 and 88, and flow monitoring means 80 and 86, are also used to monitor that proper flow is going into and out of the patient, that is there is no flow blockage such as would occur with a kinked line. Hydrophobic filters at the tops of 74 and 94 allow sterile air in and out of the reservoirs but block fluid flow. Drain pump 96 drains reservoir 94 during the fill and dwell cycles.

Pump 96 also acts as the recirculating pump during the STERILIZE/ RECIRCULATION part of the cycle. A valve 98 is a redundant (safety) element to the drain pump 96 and is also used to switch the drain flow in the STERILIZE/RECIRCULATING mode. The manual switching of the "A" output of valve 98 serves as a redundant (safety) element. The drain fluid then goes down drain 30.

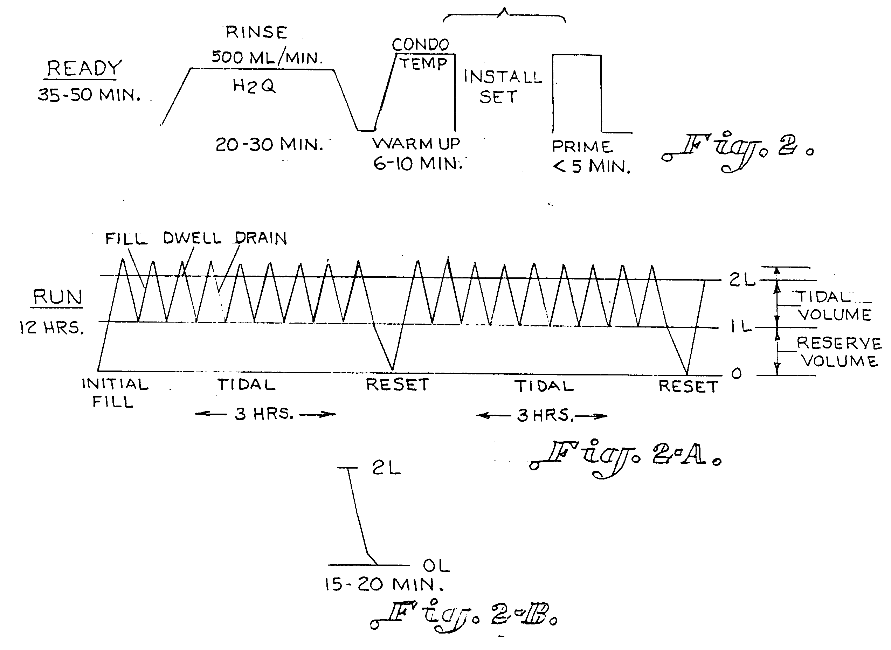

In the READY cycle, as can best be seen in Figure 2, the system is first rinsed out for at least 20 minutes to remove the formaldehyde and then pause for a test. If the test is negative, the patient then moves the concentrate, dextrose, and output lines of pump 98 to their RUN modes (safety) and proceed on into WARMUP if the computer reads that all the interlock switches are in their proper condition. The system then prepares the dialysate and fills reservoir 74 where we again go into a hold cycle while the patient is connected. The "install set" and "prime" portions of the cycle are no longer used. Then the RUN cycle is begun.

As can best be seen in Figure 2A, the peritoneal cavity is initially drained of any excess fluid and then filled to two liters (for example) and then the FILL/DRAIN or the FILL/DWELL/DRAIN portion of the cycle is started. Every N^{th} cycle, preferably N=10, the peritoneal cavity is totally drained and the actual amount is compared to that predicted. The computer then resets the fill and drain parameters to correct any significant deviation. Note that this is the method used to maintain a tidal volume in the patient, which is the difficult part of tidal peritoneal dialysis. Also, the actual ultrafiltrate is measured by weighing means 76 and 88 and the computer, and this predictive parameter is reset. At the end of the RUN cycle, the patient is completely drained.

In the STERILIZE cycle, as can best be seen in Figure 2C, the system is first cleaned with bleach for 20 minutes, the bleach rinsed out, and then the system is filled with formaldehyde, around 8% in the RO and 4% in the main flow lines and path. Then there is a switch to a recirculating mode wherein the 4% formaldehyde is circulated at 37° C or higher for six hours. The conductivity of the bleach is monitored which has a naturally conductivity of around 7.5 ms/cm at a .25% concentration, and the concentration of the formaldehyde which has had sodium chloride added to give a conductivity of 4.0 ms/cm at 4%. Both conductivities are monitored by the computer, along with the temperature (both signals coming from 70) which would alarm if the proper signal were not received for the proper time. The system then shuts totally down to wait for the READY cycle to be initiated.

In accordance with the invention, there is first a READY/RINSE cycle in order to prepare the system for the patient. During the READY/RINSE cycle, the formaldehyde is reduced to 5 ppm at the patient line. Formaldehyde from source 100 passes through valve 102 and is pumped by pump 104 to the RO membrane 20 through inlet 38. The system is drained and then flushed with RO water at 500 ml/min., alternating certain valves to catch all of the lines. The system is then held to allow time for testing for residual formaldehyde, and then the dialysis concentrate is hooked up and shifted to several other lines. The system is then warmed up with sterile, mixed, heated dialysate ready for patient connection. The continuous peritoneal dialysis process, during the patient cycle, includes the steps of initially draining the patient's peritoneal cavity of any residual fluid. Next, the patient cycle includes a fill cycle and a drain cycle. Optionally, there may be a dwell cycle between the fill and drain cycles. In the preferred embodiment, the fill and drain cycles are repeated a prescribed number of times. Afterwards, there is a total drain of the peritoneal and the total fluid in and out of the peritoneal are calculated by the computer. The system parameters are reset according to the calculations of fluid in and out for efficient dialysis, i.e. the glucose is adjusted to desired osmolality. The amount of fluid entering and leaving the patient is measured by the precision load cells on the reservoirs, backed up by the input and output flowmeters, and optionally backed up by the peritoneal volume measurement.

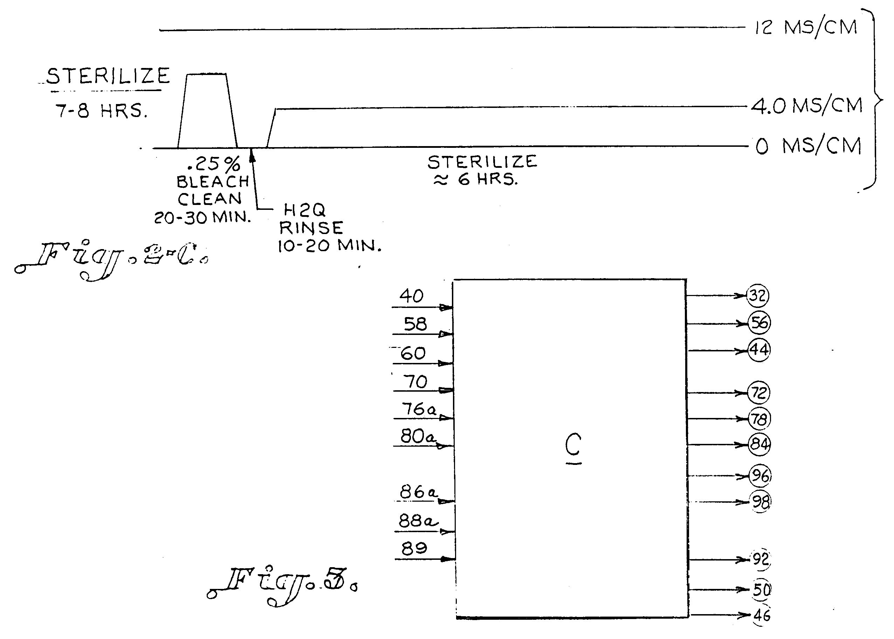

In operation, when the prescribed weight or volume of dialysis fluid is sensed by load cell 76, the valve 72 is closed. When valve 78 is opened, the heated sterilized dialysate flows through valve 78 and flowmeter 80 to the catheter 81 in the peritoneal cavity C of the patient. Valve 78 is closed as soon as a prescribed weight (i.e. volume, etc.) of the dialysate is delivered from reservoir 74. Reservoir 74 is not depleted in the run mode. It outflows to a predetermined level established by the computer. Reservoir 74 is depleted during rinse and warm-up stages. When a prescribed weight of fluid has been drained from reservoir 94, as measured by load cell 88, and valve 84 is closed and calculated by computer C. The drain cycle commences by the opening of valve 84 and fluid from the peritoneal cavity flows through valve 84 and flowmeter 86 to reservoir 94. When the system is to be reset and for the final drain, load cell 88 senses no weight change for a predetermined length of time, i.e. end of fluid draining, and the drain cycle is terminated by the closing of valve 84. The computer receives a input representing the predetermined level at 88 to control tidal volume. During the drain cycle, head vessel 74 is replenished with heated sterilized dialysate. The fill and drain cycles are then repeated. Optionally, there may be a dwell time between the end of the fill cycle and the beginning of the drain cycle. In the tidal technique, the tidal volume is assumed to be 50% of the fill volume in the preferred embodiment, but may vary in accord with the application. In the tidal peritoneal dialysis technique, there is an initial fill cycle in which the peritoneal is initially filled to a first limit, for example, 2 liters. The initial drain cycle drains the peritoneal to a reserve volume greater than zero. For example, the drain cycle may drain the patient from 2 liters to a reserve volume of 1 liter so that 1 liter remains in the patient, as can best be seen in Figure 2A. In subsequent fill and drain cycles, a tidal volume of approximately 1 liter is infused into the cavity and approximately 1 liter is drained from the cavity. In this sense, the system and method of the present invention provide an automatic tidal dialysis system. At the end of the fill/drain cycles, a complete drain of the patient occurs. The system is reset by calculating the patient weight removal, adjusting the osmolality of the dialysis fluid and again initially filling the peritoneal cavity with a prescribed amount of dialysis fluid. Other variations of this procedure may be had. The object is to achieve effective sterilization in a short time with minimal rinse out problems. The control means may be any suitable computer C such as a conventional process controller programmed to automatically compute and carry out the above described functions and control the various hardware elements illustrated in Figure 3. The peritoneal dialysis system of the invention may be used with either a single catheter 81 or a double peritoneal catheter 81a. In the case of a double catheter 81a, the inflow and outflow delivery means, 78 and 84, may be controlled by the control means so that dialysis fluid flows in and out simultaneously or alternately. While the use of inflow and outflow measuring means 80 and 86 are preferred when the double catheter is used, weighing means 76 and 88 are optional and may not be employed. Dialysis fluid may be delivered directly to the peritoneal cavity, and reservoirs 74 and 94 may be eliminated. Furthermore, flow measuring means may also be optional when using weighing means 76, 88 and reservoirs 74, 94 in the application of a single catheter. In RO unit A, the input conductivity and temperature from probe 16 and the output conductivity and temperature from probe 40 may be used to provide an alarm indicating failure in the membrane integrity if either the percent rejection or the output quality differ from set limits. In production unit D, the volumes in reservoirs 74 and 94 are measured by load cells 76 and 88. The inflow and outflow rates to the patient will be monitored either by a combination of the load cell readings and time, or flowmeters 80 and 86. In addition, either an ultrasonic or strain gauge band may be utilized to indicate the approximate peritoneal cavity volume. Probe 70 will output the conductivity and temperature of dialysate to reservoir 74 which may be diverted if the conductivity and temperature exceed plus or minus 1 ms/cm or 3° C.

## Claims

1. A system for performing a continuous peritoneal dialysis process comprising accumulation means for accumulating a sterilized dialysis fluid in a first reservoir (74); inflow delivery means (78) for delivering said dialysis fluid from said first reservoir to a peritoneal cavity of a patient; means for determining the amount of said dialysis fluid delivered to said cavity and generating a first signal (80a); outflow delivery means (84) for draining dialysis fluid from said peritoneal cavity of said patient; means for determining the amount of said dialysis fluid drained from said cavity and generating a second signal (86a); control means (C) for controlling said inflow delivery means and filling of said peritoneal cavity in response to said first signal, and for controlling said outflow delivery means and draining of said dialysis fluid from said peritoneal cavity in response to said second signal;
wherein said system is characterized by:
said control means (C) controlling said inflow delivery means (78) for initially filling said peritoneal cavity with an initial tidal, fill volume of dialysis fluid;
said control means controlling said outflow delivery means (84) after establishing said initial fill volume for partially draining a preset drain volume (V1) of said dialysis fluid from said peritoneal cavity during a drain cycle to leave a reserve volume of dialysis fluid in said peritoneal cavity which is less than said initial fill volume yet is effective for dialysis;
said control means (C) controlling said inflow means (78) after said drain cycle for refilling said peritoneal cavity with a preset fill volume of dialysis fluid during a fill cycle;
said control means (C) repeatedly controlling said inflow and outflow delivery means (78, 84) in alternating drain and fill cycles to create a tidal pool of dialysis fluid which fluctuates in volume according to said fill and drain volumes;
said drain and fill cycles being repeated in a desired number (N) of cycles defining a complete tidal dialysis regime; and
said control means controlling said outflow delivery means to totally drain dialysis fluid from said peritoneal cavity of at the end of said tidal dialysis regime to remove the ultrafiltrate from said patient.

2. The system of claim 1 including monitor means (D) for monitoring a function of the fluid volume of said peritoneal cavity and controlling said outflow delivery means to adjust the amount of dialysis fluid in said peritoneal cavity to maintain a desired fluid in said peritoneal cavity.

3. The system of claim 2 wherein said monitor means includes an ultrasonic sensing means; or a resistive sensing means.

4. The system of any one of claims 1 to 3 wherein said means for determining said amount of dialysis fluid includes a first flow measuring means (80) for monitoring a function of an inflow of said dialysis fluid to said peritoneal cavity and generating said first signal (80a) representing an amount of fluid delivered to said patent; and said control means (C) controls the amount of dialysis fluid which fills said peritoneal cavity in response to said first signal.

5. The system of claim 4 including a second flow measuring means (86) for monitoring a function of an outflow of said dialysis fluid from said peritoneal cavity and generating said second signal (86a) representing the amount of fluid drained from said patient; and said control means (C) controlling the amount of fluid which is drained from said peritoneal cavity in response to said second signal.

6. The system of any one of claims 1 to 5 wherein said control means (C) controls said inflow and outflow delivery means (78, 84) for filling and draining said dialysis fluid so as to maintain a desired reserve volume of dialysis fluid in said peritoneal cavity which is effective for dialysis.

7. The system of any one of claims 1 to 6 wherein said means for determining said amount of said inflow and outflow of dialysis fluid includes weighing means (76, 88) for weighing said inflow and outflow.

8. The system of claims 1 to 4 wherein said control means:
controls said inflow and outflow delivery means (78, 84) to repeat a number of said tidal dialysis regimes until a desired amount of water is removed from said patient.

9. The system of any one of claims 1 to 8 including monitor means (C, 90, 92) for monitoring the amount of water removed from said patient during dialysis after said total drain of each tidal dialysis regimes and adjusting an amount of glucose in said dialysis fluid to adjust the osmolality and accurately remove a prescribed weight from said patient.

10. The system of any one of the claims 1 to 9 including heater means (56) for heating said dialysis fluid prior to accumulating said first volume of dialysis fluid in said first reservoir to sterilize said dialysis fluid; storage means (62) for storing said dialysis fluid at a prescribed temperature and for a prescribed length of time prior to delivering said dialysis fluid to said first reservoir to kill the bacteria in said dialysis fluid; deaeration means (62) for deaerating said dialysis fluid prior to accumulating said dialysis fluid in said first reservoir; and measuring means (40) for measuring the conductivity of said dialysis fluid prior to delivering said dialysis fluid to said first reservoir for maintaining a prescribed conductivity.

11. The system of any one of the claims 1 to 10 wherein said control means controls (C) said inflow and said outflow delivery means (78, 84) to maintain said dialysis fluid within said peritoneal cavity for a prescribed dwell time between said filling and draining.

12. The system of claims 1 to 11 wherein said preset fill volume and preset drain volume are generally equal.

## Patentansprüche

1. Ein System zum Durchführen eines kontinuierlichen, peritonealen Dialyseverfahrens mit einer Speichereinrichtung zum Speichern einer sterilisierten Dialyseflüssigkeit in einem ersten Behälter (74), einer Zuflußfördereinrichtung (78) zum Fördern der Dialyseflüssigkeit von dem ersten Behälter zu einer Bauchhöhle eines Patienten, eine Einrichtung zum Ermitteln der in die Bauchhöhle geförderten Dialyseflüssigkeitsmenge und zum Erzeugen eines ersten Signals (80a), eine Ausflußfördereinrichtung (84) zum Abziehen der Dialyseflüssigkeit aus der Bauchhöhle des Patienten, eine Einrichtung zum Ermitteln der aus der Bauchhöhle abgezogenen Dialyseflüssigkeitsmenge und zum Erzeugen eines zweiten Signals (86a), eine Regeleinrichtung (C) zum Regeln der Zuflußfördereinrichtung und zum Befüllen der Bauchhöhle als Antwort auf das erste Signal sowie zum Regeln der Ausflußfördereinrichtung und zum Abziehen der Dialyseflüssigkeit aus der Bauchhöhle als Antwort auf das zweite Signal, wobei das System dadurch gekennzeichnet ist, daß:
die Regeleinrichtung (C) die Zuflußfördereinrichtung (78) für das anfängliche Befüllen der Bauchhöhle mit einem anschwellenden Anfangsfüllvolumen der Dialyseflüssigkeit regelt,
die Regeleinrichtung die Ausflußfördereinrichtung (84) nach dem Einfüllen des anfänglichen Füllvolumens zum teilweisen Abziehen eines voreingestellten Abziehvolumens (V1) der Dialyseflüssigkeit aus der Bauchhöhle während eines Abziehzyklus' regelt, um ein Reservevolumen an Dialyseflüssigkeit in der Bauchhöhle zurückzulassen, das kleiner ist als das anfängliche Füllvolumen und noch für die Dialyse nutzbar ist,
die Regeleinrichtung (C) die Zuflußfördereinrichtung (78) nach dem Abziehzyklus zum Wiederbefüllen der Bauchhöhle mit einem voreingestellten Füllvolumen an Dialyseflüssigkeit während eines Füllzyklus regelt,
die Regeleinrichtung (C) wiederholt die Zufluß- und Ausflußfördereinrichtung (78, 84) bei alternierenden Absaug- und Füllzyklen regelt, um eine anschwellende Ansammlung von Dialyseflüssigkeit zu schaffen, deren Volumen entsprechend den Füll- und Abziehvolumina schwankt,
die Abzieh- und Füllzyklen in einer gewünschten Anzahl (N) von Zyklen, die eine vollständige anschwellende Dialysebehandlung festlegen, wiederholt werden, und
die Regeleinrichtung die Ausflußfördereinrichtung zum vollständigen Abziehen der Dialyseflüssigkeit aus der Bauchhöhle am Ende der anschwellenden Dialysebehandlung regelt, um das Ultrafiltrat aus dem Patienten zu entfernen.

2. Das System nach Anspruch 1, das eine Überwachungseinrichtung (D) zum Überwachen einer Wirkungsweise des Flüssigkeitsvolumens der Bauchhöhle und zum Regeln der Ausflußfördereinrichtung umfaßt, um die Dialyseflüssigkeitsmenge in der Bauchhöhle zum Aufrechterhalten einer gewünschten Flüssigkeit in der Bauchhöhle anzugleichen.

3. Das System nach Anspruch 2, wobei die Überwachungseinrichtung eine Ultraschall-Erfassungseinrichtung oder eine Widerstands-Erfassungseinrichtung umfaßt.

4. Das System nach einem der Ansprüche 1 bis 3, wobei die Einrichtung zum Ermitteln der Dialyseflüssigkeitsmenge eine erste Durchflußmeßeinrichtung (80) zum Überwachen einer Wirkungsweise eines Zuflusses der Dialyseflüssigkeit in die Bauchhöhle und zum Erzeugen des ersten Signals (80a), das eine in den Patienten geförderte Flüssigkeitsmenge darstellt, umfaßt, und wobei die Regeleinrichtung (C) die Dialyseflüssigkeitsmenge, die die Bauchhöhle als Antwort auf das erste Signal füllt, regelt.

5. Das System nach Anspruch 4, das eine zweite Durchflußmeßeinrichtung (86) zum Überwachen einer Wirkungsweise eines Ausflusses der Dialyseflüssigkeit aus der Bauchhöhle und zum Erzeugen des zweiten Signals (86a), das die aus dem Patienten abgezogene Flüssigkeitsmenge darstellt, umfaßt, und wobei die Regeleinrichtung (C) die aus der Bauchhöhle als Antwort auf das zweite Signal abgezogene Flüssigkeitsmenge regelt.

6. Das System nach einem der Ansprüche 1 bis 5, wobei die Regeleinrichtung (C) die Zufluß- und Ausflußfördereinrichtung (78, 84) zum Füllen und Abziehen der Dialyseflüssigkeit regelt, so daß ein erwünschtes Reservevolumen an Dialyseflüssigkeit in der Bauchhöhle gehalten wird, das für die Dialyse nutzbar ist.

7. Das System nach einem der Ansprüche 1 bis 6, wobei die Einrichtung zum Ermitteln der Menge des Zuflusses und Ausflusses der Dialyseflüssigkeit Wägeeinrichtungen (76, 88) zum Wiegen des Zuflusses und Ausflusses umfaßt.

8. Das System nach Anspruch 1 bis 4, wobei die Regeleinrichtung die Zufluß- und Ausflußfördereinrichtung (78, 84) regelt, um eine Anzahl von anschwellenden Dialysebehandlungen zu wiederholen, bis eine gewünschte Menge von Wasser aus dem Patienten entfernt ist.

9. Das System nach einem der Ansprüche 1 bis 8, das Überwachungseinrichtungen (C, 90, 92) zum Überwachen der aus dem Patienten entfernten Wassermenge während der Dialyse nach dem vollständigen Abziehen bei einer jeden anschwellenden Dialysebehandlung und zum Angleichen einer Glukosemenge in der Dialyseflüssigkeit umfaßt, um die Osmolalität anzugleichen und ein vorgeschriebenes Gewicht von dem Patienten genauestens zu entfernen.

10. Das System nach einem der Ansprüche 1 bis 9, das eine Heizeinrichtung (56) zum Erwärmen der Dialyseflüssigkeit vor dem Sammeln des ersten Dialyseflüssigkeitsvolumens in dem ersten Behälter zum Sterilisieren der Dialyseflüssigkeit, eine Speichereinrichtung (62) zum Speichern der Dialyseflüssigkeit bei einer vorgeschriebenen Temperatur und für eine vorgeschriebene Zeitdauer, um die Dialyseflüssigkeit zum Abtöten der Bakterien in der Dialyseflüssigkeit zu dem ersten Behälter zu fördern, eine Entlüftungseinrichtung (62) zum Entlüften der Dialyseflüssigkeit vor dem Sammeln der Dialyseflüssigkeit in dem ersten Behälter, und eine Meßeinrichtung (40) zum Messen der Leitfähigkeit der Dialyseflüssigkeit vor dem Fördern der Dialyseflüssigkeit in den ersten Behälter zum Aufrechterhalten einer vorgeschriebenen Leitfähigkeit umfaßt.

11. Das System nach einem der Ansprüche 1 bis 10, wobei die Regeleinrichtung (C) die Zufluß- und Ausflußfördereinrichtung (78, 84) regelt, um die Dialyseflüssigkeit innerhalb der Bauchhöhle für eine vorgeschriebene Verweilzeit zwischen dem Füllen und dem Abziehen zu halten.

12. Das System nach Anspruch 1 bis 11, wobei das voreingestellte Füllvolumen und das voreingestellte Abziehvolumen im wesentlichen gleich sind.

## Revendications

1. Système pour l'exécution d'une opération de dialyse péritonéale continue, comprenant un moyen d'accumulation pour accumuler un liquide de dialyse stérilisé dans un premier réservoir (74); un moyen débiteur d'entrée (78) pour délivrer ledit liquide de dialyse à partir dudit premier réservoir à la cavité péritonéale d'un patient; un moyen pour déterminer la quantité dudit liquide de dialyse délivrée à ladite cavité et émettre un premier signal (80a); un moyen débiteur de sortie (84) pour évacuer le liquide de dialyse à partir de ladite cavité péritonéale dudit patient; un moyen pour déterminer la quantité dudit liquide de dialyse évacuée de ladite cavité et émettre un second signal (86a); un moyen de commande (C) pour commander ledit moyen débiteur d'entrée et contrôler le remplissage de ladite cavité péritonéale en réponse audit premier signal, et pour commander ledit moyen débiteur de sortie et contrôler l'évacuation dudit liquide de dialyse à partir de ladite cavité péritonéale en réponse audit second signal,
caractérise en ce que
ledit moyen de commande (C) pilote ledit moyen débiteur d'entrée (78) de façon à remplir initialement ladite cavité péritonéale d'un volume de remplissage de liquide de dialyse en un afflux initial;
ledit moyen de commande pilote ledit moyen débiteur de sortie (84), après l'établissement dudit volume de remplissage initial, de façon à évacuer partiellement un volume de drainage prédéfini (V1) dudit liquide de dialyse à partir de ladite cavité péritonéale, au cours d'un cycle de drainage, en laissant dans ladite cavité péritonéale un volume de réserve de liquide de dialyse qui est plus petit que ledit volume de remplissage initial, mais est néanmoins suffisant pour une dialyse efficace;
ledit moyen de commande (C) pilote ledit moyen d'entrée (78), après ledit cycle de drainage, de façon à remplir de nouveau ladite cavité péritonéale d'un volume de remplissage prédéfini de liquide de dialyse au cours d'un cycle de remplissage;
ledit moyen de commande (C) pilote à répétition lesdits moyens débiteurs d'entrée et de sortie (78,84), dans des cycles alternants de drainage et ne remplissage, pour créer un afflux de liquide de dialyse dont le volume fluctue conformément auxdits volumes de remplissage et de drainage;
lesdits cycles de drainage et de remplissage sont répétés en un nombre désiré (N) de cycles définissant un traitement complet de dialyse par afflux; et
ledit moyen de commande pilote ledit moyen débiteur de sortie de façon à évacuer totalement le liquide de dialyse de ladite cavité péritonéale, à la fin dudit traitement de dialyse par afflux, pour débarrasser ledit patient du produit d'ultrafiltration.

2. Système selon la revendication 1, comprenant un moyen de contrôle (D) pour surveiller une fonction du volume de fluide de ladite cavité péritonéale et commander ledit moyen débiteur de sortie de façon à régler la quantité de liquide de dialyse dans ladite cavité péritonéale assurant le maintien d'un liquide désiré dans ladite cavité péritonéale.

3. Système selon la revendication 2, dans lequel ledit moyen de contrôle comprend un moyen détecteur à ultrasons ou un moyen détecteur à résistance.

4. Système selon l'une quelconque des revendications 1 à 3, dans lequel ledit moyen de détermination de ladite quantité de liquide de dialyse comprend un premier moyen de mesure de débit (80) pour surveiller une fonction d'un afflux d'entrée dudit liquide de dialyse vers ladite cavité péritonéale et émettre ledit premier signal (80a) représentant la quantité de liquide délivrée audit patient; et ledit moyen de commande (C) régit la quantité de liquide de dialyse qui remplit ladite cavité péritonéale en réponse audit premier signal.

5. Système selon la revendication 4, comprenant un second moyen de mesure de débit (86) pour surveiller une fonction d'un afflux de sortie dudit liquide de dialyse à partir de ladite cavité péritonéale et émettre ledit second signal (86a) représentant la quantité de liquide évacuée dudit patient; et ledit moyen de commande (C) régit la quantité de liquide qui est évacuée de ladite cavité péritonéale en réponse audit second signal.

6. Système selon l'une quelconque des revendications 1 à 5, dans lequel ledit moyen de commande (C) pilote lesdits moyens débiteurs d'entrée et de sortie (78,84) de façon a introduire et à évacuer ledit liquide de dialyse de manière à maintenir, dans ladite cavité péritonéale, un volume de réserve désiré de liquide de dialyse qui est suffisant pour une dialyse efficace.

7. Système selon l'une quelconque des revendications 1 à 6, dans lequel lesdits moyens de détermination de ladite quantité desdits afflux d'entrée et de sortie de liquide de dialyse comprennent des moyens de pesée (76,88) pour peser lesdits afflux d'entrée et de sortie.

8. Système selon l'une quelconque des revendications 1 à 4, dans lequel ledit moyen de commande pilote lesdits moyens débiteurs d'entrée et de sortie (78,84) de façon à répéter un certain nombre desdits traitements de dialyse par afflux jusqu'à ce qu'une quantité désirée d'eau soit extraite dudit patient.

9. Système selon l'une quelconque des revendications 1 à 8, comprenant des moyens de contrôle (C,90,92) pour surveiller la quantité d'eau extraite dudit patient au cours de la dialyse après ladite évacuation totale de chaque traitement de dialyse par afflux et régler, dans ledit liquide de dialyse, une quantité de glucose propre à régler l'osmolalité et à extraire avec précision dudit patient un poids prescrit.

10. Système selon l'une quelconque des revendications 1 à 9, comprenant un moyen chauffant (56) pour chauffer ledit liquide de dialyse avant l'accumulation dudit premier volume de liquide de dialyse dans ledit premier réservoir afin de stériliser ledit liquide de dialyse; un moyen de stockage (62) pour stocker ledit liquide de dialyse à une température prescrite et pendant une période de temps prescrite, avant de l'envoyer dans ledit premier réservoir, pour tuer les bactéries contenues dans ledit liquide de dialyse; un moyen de désaération (62) pour désaérer ledit liquide de dialyse avant son accumulation dans ledit premier réservoir; et un moyen de mesure (40) pour mesurer la conductivité dudit liquide de dialyse avant qu'il ne soit envoyé dans ledit premier réservoir, afin de maintenir une conductivité prescrite.

11. Système selon l'une quelconque des revendications 1 à 10, dans lequel ledit moyen de commande (C) pilote lesdits moyens débiteurs d'entrée et de sortie (78,84) de façon à maintenir ledit liquide de dialyse dans ladite cavité péritonéale pendant une durée de séjour prescrite entre ledit remplissage et ledit drainage.

12. Système selon l'une quelconque des revendications 1 à 11, dans lequel ledit volume de remplissage prédéfini et ledit volume de drainage prédéfini sont généralement égaux.
